(19) **Europäisches Patentamt**
**European Patent Office**
**Office européen des brevets**

(11) **EP 2 450 003 B1**

(12) **EUROPÄISCHE PATENTSCHRIFT**

(45) Veröffentlichungstag und Bekanntmachung des Hinweises auf die Patenterteilung:
**30.05.2018  Patentblatt 2018/22**

(51) Int Cl.:
***A61C 13/00*** (2006.01)

(21) Anmeldenummer: **11188041.5**

(22) Anmeldetag: **07.11.2011**

(54) **Verfahren zur Herstellung eines Grünkörpers sowie vorgesinteter Grünkörper**

Method for manufacturing a blank and pre-sintered blank

Procédé de fabrication d'une ébauche et ébauche pré-frittée

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.11.2010  EP 10190512**

(43) Veröffentlichungstag der Anmeldung:
**09.05.2012  Patentblatt 2012/19**

(73) Patentinhaber: **DENTSPLY SIRONA Inc.**
**York, PA 17401-2991 (US)**

(72) Erfinder:
- **Hachenberg, Jörg**
  **63739 Aschaffenburg (DE)**
- **Steinke, Rudi**
  **63457 Hanau (DE)**
- **Vollmann, Markus**
  **63571 Gelnhausen (DE)**
- **Wissel, Irmgard**
  **63579 Freigericht (DE)**
- **Zellmann, Gerhard**
  **63589 Linsengericht (DE)**
- **Hock, Elmar**
  **63776 Mömbris (DE)**
- **Fecher, Stefan**
  **63867 Johannesberg (DE)**
- **Völkl, Lothar**
  **63773 Goldbach (DE)**

(74) Vertreter: **Stoffregen, Hans-Herbert**
**Patentanwalt**
**Friedrich-Ebert-Anlage 11b**
**63450 Hanau (DE)**

(56) Entgegenhaltungen:
**AT-A1- 505 698        US-A- 4 996 022**
**US-A1- 2005 023 710**

- **WILSON CORRÊA RODRIGUES ET AL: "Powder metallurgical processing of Co-28%Cr-6%Mo for dental implants: Physical, mechanical and electrochemical properties", POWDER TECHNOLOGY, ELSEVIER SEQUOIA, LAUSANNE, CH, Bd. 206, Nr. 3, 29. September 2010 (2010-09-29), Seiten 233-238, XP002632108, ISSN: 0032-5910, DOI: 10.1016/J.POWTEC.2010.09.024 [gefunden am 2010-09-29]**

**Beschreibung**

Die Erfindung bezieht sich auf ein Verfahren zur Herstellung eines Formkörpers, insbesondere eines Zahnersatzes oder eines Teiles von diesem, durch Mischen von einem Metallpulver.

In jüngerer Zeit wird zur Herstellung von Zahnersatz wie Kronen oder Brücken die CAD/CAM-Technik (Computer-Aided Design, Computer Aided Manufacturing) verwendet, die insbesondere auf dem Gebiet der Keramik zum Einsatz gelangt. Beispielhaft ist auf die EP-B-1 067 880 zu verweisen.

Der DE-C-199 38 144 ist ein Verfahren zur Herstellung von Zahnersatz zu entnehmen, wobei ein aus einer Keramik bestehender vorgesinterter Rohling durch fräsendes Verfahren bearbeitet und sodann dichtgesintert wird.

Aus der WO-2009/120749 ist ein CAD/CAM-Fräsverfahren bekannt, um einen Zahnersatz herzustellen. Hierzu wird zunächst ein Metallpulver mit einem Bindemittel gemischt, um sodann durch Metallpulverspritzgießen einen Rohling herzustellen. Aus diesem wird durch fräsende Bearbeitung ein Formkörper hergestellt, der dem herzustellenden Zahnersatz unter Berücksichtigung der beim Sintern des Rohlings auftretenden Schrumpfung entspricht.

Nach der EP-A-1 764 062 wird ein Formkörper aus einer Dentallegierung hergestellt, der aus einem durch heißisostatisches Pressen dichtgesinterten Dentallegierungspulver besteht.

Aus der DE-A-103 52 231 ist es bekannt, einen Formkörper pulvermetallurgisch herzustellen, wobei der Körper zum Bearbeiten offenporig und nicht dichtgesintert ist. Erst nach Herstellung der Endform werden in einem weiteren Arbeitsschritt mittels Infiltrationsverfahren die offenen Poren des Formkörpers mit einer zweiten Legierung geschlossen. Nachteilig ist der Einsatz zweier Legierungen.

Die US-A-2005/0023710 bezieht sich auf ein Verfahren zur Herstellung eines Formkörpers in Form eines Zahnersatzes. Dabei gelangt ein additives Herstellungsverfahren zur Anwendung. Um in Freiformtechnik den Zahnersatz herzustellen, wird auf der Basis von CAD-Daten eine vergrößerte Form des Zahnersatzes hergestellt, wobei schichtweise Mischungen aus Metallpulver und Bindemittel aufgetragen werden. Als Metallpulver kommt insbesondere ein solches aus der Gruppe Gold, Metalle der Platin-Gruppe, Silber und Legierung dieser in Frage. Die mittels des additiven Verfahrens hergestellte Form ist gegenüber dem einzusetzenden Zahnersatz vergrößert und zwar um den Faktor der Schrumpfung, die beim anschließenden Sintern auftritt.

In der Literaturstelle Rodrigues et al.: "Powder metallurgical Processing of Co-28%Cr-6%Mo for dental implants: Physical, cheanical and electrochemical properties" Powder Technology, 2006 (2011), 233-238, wird ein Verfahren zur Herstellung von Restaurationen beschrieben. Hierzu wird eine biokompatible Kobalt-Chrom-Molybdän-Legierung mit einem Gleitmittel gemischt, auf eine Temperatur zum Ausbrennen des Gleitmittels erwärmt und sodann durchgesintert.

Aus der US-A-4,996,022 ist ein Verfahren zur Herstellung eines Sinterkörpers bekannt. Als Ausgangsmaterial wird ein Metallpulver wie Eisen oder Nickel benutzt.

Eine Eisenpulver-Mischung, die bis zu 1 % organisches Bindemittel enthält, wird zur Herstellung eines Sinterformteils nach der AT-A-505 698 benutzt. Nach einem Vorsintern und Abkühlen wird ein Endsintern durchgeführt.

Der vorliegenden Erfindung liegt die Aufgabe zu Grunde, ein Verfahren und einen vorgesinterten Grünkörper der eingangs genannten Art so weiterzubilden, dass ein Formkörper zur Verfügung gestellt werden kann, insbesondere ein Zahnersatz oder ein Teil von diesem, der mit überaus geringer Toleranz herstellbar und problemlos nass oder trocken zu bearbeiten ist, wobei insbesondere auch die Möglichkeit gegeben sein soll, diesen mit einem Keramikmaterial zu verblenden. Dabei sollen die Nachteile des Standes der Technik vermieden werden.

Auch soll ein Grünling zur Verfügung gestellt werden, der auf einfache Weise mit hoher Genauigkeit bearbeitet werden kann, um sodann ein hochpräzises Formteil, insbesondere einen Zahnersatz oder ein Teil von diesem herstellen zu können.

Erfindungsgemäß wird die Aufgabe im Wesentlichen durch ein Verfahren zur Herstellung eines Formkörpers, insbesondere eines Zahnersatzes oder eines Teils von diesem, mit den Verfahrensschritten gelöst:

- Herstellung einer Mischung aus einem Metallpulver in Form einer Nickel-Chrom-Legierung oder einer Kobalt-Chrom-Legierung und einem Bindemittel,
- Verdichten der Mischung zu einem Grünling,
- Aufheizen des Grünlings von Zimmertemperatur auf eine Entbinderungsstarttemperatur $T_1$,
- Entbindern des Grünlings, wobei der Grünling zum Entbindern zunächst auf die Entbinderungsstarttemperatur $T_1$ mit 350 °C $\leq T_1 \leq$ 550 °C erhitzt und sodann im Temperaturbereich zwischen $T_1$ und der Entbinderungsendtemperatur $T_2$ mit 550 °C < $T_2$ < 650 °C mit $T_2 > T_1$ bei einer Aufheizrate $R_1$ mit 1 K/min $\leq R_1 \leq$ 5 K/min auf $T_2$ erhitzt wird,
- Vorsintern des entbinderten Grünlings, wobei der Grünling mit einer Aufheizrate $R_{HVS}$ mit 5 K/min $\leq R_{HVS} \leq$ 200 K/min auf eine Vorsinterendtemperatur $T_{VS}$ mit 750 °C $\leq T_{VS}$ < 850 °C erhitzt und über eine Haltezeit $T_{vs}$, mit 5 Min. $\leq T_{vs} \leq$ 60 Min. gehalten wird,
- Kühlen des Grünlings von der Vorsinterendtemperatur $T_{VS}$ mit einer Kühlrate $R_{KVS}$ mit 5 K/min $\leq R_{KVS} \leq$ 200 K/min, wobei zumindest die Aufheizrate $R_{HVS}$, die Vorsinterendtemperatur $T_{VS}$ und die Kühlrate $R_{KVS}$ derart aufeinander abgestimmt werden, dass der vorgesinterte, einen Rohling bildende Grünling eine Flächenporosität zwischen 16 % und 22 % nach dem Vorsintern aufweist,

- abtragendes Bearbeiten des Rohlings und
- Dichtsintern des bearbeiteten Rohlings zur Bildung des Formkörpers.

Unter Flächenporosität wird hierbei der Anteil der Fläche bezeichnet, welcher bei Anfertigung eines metallographischen Schliffes nicht mit Material gefüllt ist.

Bei einer Kobalt-Chrom-Legierung sollte eine Zusammensetzung wie folgt gewählt werden:

| | |
|---|---|
| Kobalt: | 50 Gew.-% bis 70 Gew.-% |
| Chrom: | 20 Gew.-% bis 35 Gew.-% |
| Molybdän: | 0 Gew.-% bis 10 Gew.-% |
| Wolfram: | 0 Gew.-% bis 20 Gew.-% |
| weitere Elemente: | weniger als 10 Gew.-%, |

wobei die Gesamtsumme 100 Gew.-% beträgt.

Auch besteht die Möglichkeit, eine Nickel-Chrom-Legierung der Zusammensetzung zu verwenden:

| | |
|---|---|
| Nickel: | 50 Gew.-% bis 70 Gew.-% |
| Chrom: | 20 Gew.-% bis 35 Gew.-% |
| Molybdän: | 0 Gew.-% bis 10 Gew.-% |
| Wolfram: | 0 Gew.-% bis 20 Gew.-% |
| weitere Elemente: | weniger als 10 Gew.-%, |

mit einer Gesamtsumme von 100 Gew.-%.

Als weitere Elemente kommen insbesondere Mangan, Silizium, Nickel bei der Kobalt-Chrom-Legierung, Kobalt bei der Nickel-Chrom-Legierung, Beryllium, Cadmium, Blei, Eisen, Aluminium, Titan, Kohlenstoff, Stickstoff, Sauerstoff, Schwefel sowie sonstige Elemente mit einem Gewichtsanteil von weniger als 1 % in Frage.

Insbesondere ist vorgesehen, dass die zu dem Grünling verdichtete Mischung eine Flächenporosität, die der Volumenporosität entspricht, zwischen 16 % und 27 %, vorzugsweise zwischen 18 % und 22 % aufweist. Die Porosität wird dabei durch die Bereiche des Grünlings gebildet, die zwischen den Metallpulverpartikeln mit Luft oder Bindemittel gefüllt sind.

Insbesondere wird auch vorgeschlagen, dass der Grünling mit der Kühlrate $R_{KVS}$ auf eine Temperatur $T_3$ abgekühlt wird, wobei insbesondere $T_3 \leq T_2$, 450 °C. $\leq T_3 \leq$ 650 °C, bevorzugterweise $T_3$ in etwa 600 °C ist.

Das Entbindern und Vorsintern sollte dabei unter Sauerstoffabschluss, insbesondere in einer Inert- oder besonders bevorzugt Argonatmosphäre erfolgen. Auch eine reduzierende Atmosphäre oder Vakuum kommen in Frage.

Zum Entbindern wird der Grünling auf eine Entbinderungsstarttemperatur $T_1$ mit 350 °C $\leq T_1 \leq$ 550 °C erhitzt. Nach Erreichen der Temperatur $T_1$, insbesondere dem Erreichen der Temperatur $T_1 \cong$ 450 °C erfolgt sodann ein langsames Aufheizen, wobei die Aufheizrate beträgt 1 K/min bis 5 K/min. Insbesondere ist vorgesehen, dass im Bereich oberhalb 500 °C, insbesondere oberhalb 550 °C bis zur Entbinderungsendtemperatur $T_2$ mit 550 °C $\leq T_2 \leq$ 650 °C, insbesondere $T_2 \cong$ 600 °C, eine Aufheizrate zwischen insbesondere 1 K/min und 5 K/min gewählt wird. Nach Erreichen der Entbinderungsendtemperatur $T_2$ sollte sodann der Grünling über eine Zeit $t_2$ mit 1 min $\leq t_2 \leq$ 20 min gehalten werden. Dies ist jedoch nicht zwingend erforderlich und im Wesentlichen von der Aufheizrate abhängig.

Unabhängig von den bevorzugten, jedoch beispielhaft und nicht schutzeinschränkend angegebenen Parametern muss das Aufheizen dabei derart erfolgen, dass das Entbindern kontrolliert erfolgt, wodurch anderenfalls der Grünling beschädigt und somit unbrauchbar würde. Dieses kontrollierte Aufheizen, ohne dass der Grünling beschädigt wird, kann ein Durchschnittsfachmann gegebenenfalls nach Durchführung einfacher Versuche ohne Weiteres durchführen.

Nach dem Entbindern erfolgt ein Aufheizen auf die Vorsinterendtemperatur Tvs, wobei grundsätzlich beliebige Heizraten $R_{HVS}$ gewählt werden können.

Um die gewünschte Flächenporosität des vorgesinterten Grünlings zwischen 16 % und 22 %, insbesondere zwischen 18 % und 20 % zu erzielen, erfolgt erfindungsgemäß eine Abstimmung von Vorsinterendtemperatur $T_{VS}$, Aufheizrate $R_{HVS}$, gegebenenfalls Haltezeit $t_{VS}$ bei der Vorsinterendtemperatur $T_{VS}$ und Kühlrate $R_{KVS}$. Bei einem sehr langsamen Aufheizen auf die Vorsinterendtemperatur $T_{VS}$, z. B. mit einer Aufheizrate zwischen 1 K/min und 10 K/min, ist es nicht erforderlich, dass der entbinderte Grünling bei der Vorsinterendtemperatur über eine Zeit $t_{VS}$ gehalten wird.

Auch durch die Kühlrate kann eine Beeinflussung der Haltezeit tvs erfolgen, und zwar bis zu einem Umfang, dass nach Erreichen der Vorsinterendtemperatur $T_{VS}$ unmittelbar ein Kühlen erfolgt.

Die Abstimmung der Parameter zur Erreichung der gewünschten Flächen- bzw. Volumenporosität zwischen 16 % und 22 %, insbesondere zwischen 18 % und 20 %, erfolgt unter Berücksichtigung nachstehender Angaben.

Bei Vorliegen der Vorsinterendtemperatur $T_{VS}$ zwischen 750 °C und 850 °C sollte die Aufheizrate $R_{HVS}$ und/oder die Kühlrate $R_{KVS}$ zwischen 5 K/min und 200 K/min, insbesondere zwischen 5 K/min und 20 K/min liegen. Nach Erreichen der Vorsinterendtemperatur $T_{VS}$ ist eine Haltezeit $t_{VS}$ zwischen 5 min und 60 min, insbesondere zwischen 10 min und 30 min zu wählen.

Es können unterschiedliche Aufheizraten, Vorsinterendtemperaturen, Haltezeiten und Kühlraten gewählt werden, die derart aufeinander abzustimmen sind, dass sich eine Flächenporosität des vorgesinterten Grünlings, der auch als Rohling zu bezeichnen ist, mit einer Flächenporosität zwischen 16 % und 22 % ergibt.

Insbesondere sollte eine Abstimmung der Parameter derart erfolgen, dass sich eine Flächenporosität zwischen 18 % und 20 % ergibt.

Insbesondere ist vorgesehen, dass der Grünling vor dem Entbindern eine Porosität aufweist, die maximal 5 % über der Flächenporosität nach dem Vorsintern liegt. Insbesondere sollte die Differenz weniger als 2 % betragen.

Daher zeichnet sich die Erfindung auch dadurch aus, dass ein verdichteter Grünling zur Herstellung des Formkörpers verwendet wird, dessen Porosität zwischen 16 % und 27 %, insbesondere zwischen 18 % und 22 % liegt.

Liegt die Porosität des verdichteten, jedoch nicht entbinderten Grünlings grundsätzlich oberhalb der Porosität des vorgesinterten Grünlings, also des Rohlings, so kann ebenfalls die Porosität gleich sein, ohne dass die Erfindung verlassen wird.

Es besteht eine Korrelation zwischen Heizrate, Vorsinterendtemperatur, Haltezeit und Kühlrate. So ist bei niedrigeren Heiz- und Kühlraten eine kürzere Haltezeit einzustellen und umgekehrt. Alle Raten und Haltezeiten werden maßgeblich durch die Wahl der Vorsinterendtemperatur bestimmt. Haltezeiten unter 5 min sind weniger geeignet, da insbesondere bei größeren Rohlingen bei kürzeren Haltezeiten eine homogene Durchwärmung und Vorsinterung nicht gewährleistet werden können. Haltezeiten von über 60 min sind ebenfalls unvorteilhaft, da eine längere Verweilzeit die unerwünschte Bildung einer Oxidationsschicht begünstigt.

Formkörper mit einer entsprechenden Flächenporosität zeigen ein überaus gutes Bearbeitungsverhalten, um insbesondere einen Zahnersatz oder Zahnersatzteil herstellen zu können. Es kann eine präzise Bearbeitung bei geringem Werkzeugverschleiß erfolgen.

Nach Abkühlen des Rohlings auf Zimmertemperatur erfolgt sodann die abtragende Bearbeitung zum Formkörper, wobei Fräsen und Schleifen zu nennen sind.

Schließlich wird der Schritt des Dichtsinterns durchgeführt.

Als Metallpulver wird ein solches auf Nickel-Chrom-Basis oder auf Kobalt-Chrom-Basis, insbesondere ein Dentallegierungspulver in Form einer Kobalt-Chrom-Legierung, vorzugsweise Kobalt-Chrom-Molybdän-Legierung verwendet.

Als Bindemittel ist bevorzugterweise ein solches anzugeben, das auf Wachs- und/oder Zellulose-Basis beruht.

Insbesondere ist vorgesehen, dass zur Erzielung einer Flächenporosität zwischen 16 % und 22 %, insbesondere zwischen 18 % und 20 %, der Grünling bei der Vorsinterendtemperatur Tvs über eine Zeit $t_{VS}$ gehalten wird gemäß der Beziehung

$$t/2 < t_{VS} < 2t \qquad\qquad (1).$$

Dabei berechnet sich t nach der Gleichung:

$$t = t_0 \cdot \ln\left(\frac{c_0}{c}\right) \cdot \exp\left(\frac{T_0}{T_{VS}}\right) \qquad\qquad (2)$$

mit

c = Gewünschter Flächenporositätsanteil des Grünlings nach dem Vorsintern,
$c_0$ = Flächenporositätsanteil des Grünling nach dem Entbindern,
$t_0$ = Materialkonstante in min.,
$T_0$ = Materialkonstante in Kelvin,
$T_{VS}$ = Vorsinterendtemperatur bei der Haltezeit $t_{VS}$ mit 650 °C ≤ $T_{VS}$ ≤ 1100 °C.

$c_0$, also der Flächenporositätsanteil des Grünlings nach dem Entbindern, kann durch Interpolation von Messergebnissen bestimmt werden, wobei grundsätzlich die Beziehung gilt $c_0 - c < 5$ %, insbesondere $c_0 - c < 2$ %. Als weitere Nebenbedingung ist anzugeben: $c < c_0$.

Auch die Materialkonstante $t_0$ kann durch Interpolation von Messergebnissen bestimmt werden. Bei Verwendung von Metallpulver auf Kobalt-Chrom-Basis oder äquivalenten Materialien ergibt sich $t_0 = 0,0125$ min.

Die Materialkonstante $T_0$ beträgt bei der Verwendung von Metallpulver auf Kobalt-Chrom-Basis oder Äquivalenten hierzu $T_0 = 11000$ K.

Es ist zu beachten, dass Tvs in obige Formel in Kelvin und nicht in Grad Celsius einzusetzen ist.

Erfolgt das Vorsintern gemäß dieses Zusammenhanges durch Halten der Vorsinterendtemperatur über eine Haltezeit $t_{VS}$, so sind Kühl- und Aufheizraten so kurz zu wählen, dass der Hauptteil des Vorsinterns während der Haltezeit erfolgt. Insbesondere sollten beim Aufheizen mit annähernd konstanter Aufheizrate $R_{HVS}$ und Kühlen mit annähernd konstanter Kühlrate $R_{KVS}$ die Aufheizzeit zwischen 650°C und der Vorsinterendtemperatur $T_{VS}$ und die Kühlzeit zwischen Vorsinterendtemperatur $T_{VS}$ und 650°C in Summe zusammen nicht länger sein als 2t:

$$\frac{T_{VS} - 650°C}{R_{HVS}} + \frac{T_{VS} - 650°C}{R_{KVS}} < 2t, \qquad (3)$$

wobei Temperaturen in Grad Celsius einzusetzen sind. Ferner ist die Nebendingung einzuhalten, dass die Vorsinterendtemperatur $T_{VS}$ und die Differenz zwischen $c_0$ und c derart aufeinander abgestimmt sind, dass eine negative Aufheiz- und Abkühlrate ausgeschlossen ist. Auch sollte die maximale Vorsinterendtemperatur $T_{VS}$ nicht größer als 1100 °C betragen.

Die Formel (3) stellt folglich eine Bedingung dar, die bei Zugrundelegung der angegebenen Parameter einzuhalten ist, um bei Verwendung eines Metallpulvers auf Kobalt-Chrom-Basis oder Äquivalenten hierzu einen vorgesinterten Rohling herzustellen, der eine Flächenporosität zwischen 16 % und 22 % aufweist.

Überraschenderweise hat sich herausgestellt, dass ein vorgesinterter Rohling ungeachtet der bestehenden Flächenporosität nicht nur mit der gewünschten Genauigkeit bearbeitet werden kann, sondern dass außerdem nach dem Dichtsintern ungeachtet der Restflächenporosität eine absolut blasenfreie Verblendung möglich ist. Ursächlich hierfür dürfte sein, dass bei den erfindungsgemäßen Vorsinterschritten unter Berücksichtigung der angegebenen Parameter eine Restflächenporosität erzielbar ist, die nach dem Dichtsintern kein verbundenes System darstellt, sondern vereinzelt vorliegt. Hierdurch wird nicht nur erwähntermaßen die Möglichkeit einer blasenfreien keramischen Verblendung geboten, sondern auch die erforderliche Korrosionsbeständigkeit sichergestellt. Ferner konnte festgestellt werden, dass nach dem Bearbeiten nach dem darauf folgenden Dichtsintern die erforderliche Maßhaltigkeit erzielbar ist, also eine gleichmäßige und passungsgerechte Schrumpfung erfolgt. In Weiterbildung der Erfindung ist vorgesehen, dass die Mischung aus Legierungspulver und Bindemittel axial oder isostatisch bei einem Druck p mit 100 MPa $\leq$ p $\leq$ 1.000 MPa, insbesondere mit 200 MPa $\leq$ p $\leq$ 600 MPa, gepresst werden sollte.

Ferner ist bevorzugterweise vorgesehen, dass der Grünling bzw. der entbinderte Grünling als auch der entbinderte und vorgesinterte Grünling in einer Inert- oder Formiergas-Atmosphäre oder im Vakuum aufgeheizt werden. Durch diese Maßnahmen ist sichergestellt, dass oberflächlich eine nur sehr dünne Oxidschicht entsteht, die z. B. durch Polieren problemlos entfernbar ist, ohne dass die Restflächenporosität die Politur nach dem Dichtsintern beeinträchtigt.

Der so hergestellte Rohling kann sodann insbesondere durch ein trocken oder nass arbeitendes Werkzeug bearbeitet werden, wobei insbesondere in CAM-Technik aus dem Rohling entsprechend dessen Dimensionierung eine gewünschte Anzahl von Formkörpem, insbesondere von Zahnersatz wie Kronen oder Brücken, insbesondere fräsend oder schleifend herausgearbeitet werden. Ein Kopierfräsen ist auch möglich.

Weitere Einzelheiten, Vorteile und Merkmale ergeben sich nicht nur aus den Ansprüchen, den diesen zu entnehmenden Merkmalen, sondern auch aus der nachfolgenden Beschreibung bevorzugter Ausführungsbeispiele.

Zur Herstellung von Zahnersatz wurde eine Metalllegierung der Zusammensetzung

26 bis 30 Gew.-% Cr,
5 bis 7 Gew.-% Mo,
insgesamt zwischen 0,01 und 1,5 Gew.-% zumindest eines
der Elemente Mn, Si, Fe, C, Ni,
Rest Co (61,5 Gew.-% bis 68,99 Gew.-%)

benutzt, wobei die Gesamtsumme 100 Gew.-% beträgt. Zur Herstellung des Pulvers wurde zunächst eine Metalllegierung hergestellt, erschmolzen und anschließend verdüst. Die mittlere Korngröße lag zwischen 5 $\mu$m und 50 $\mu$m. Anschließend wurden ca. 2 Gew.-%, bezogen auf das Metallpulver, ein Binder auf Wachsbasis zugegeben. Mit der so hergestellten Mischung wurden mittels axialem Pressen Grünlinge hergestellt, die eine Scheibenform aufweisen. Der Durchmesser betrug in etwa 10 cm und die Dicke in etwa 1 cm. Andere Dimensionierungen kommen gleichfalls in Frage.

Anschließend erfolgte ein Entbindern. Hierzu wurden Grünlinge zunächst mit beliebiger Aufheizrate auf 450 °C aufgeheizt. Oberhalb 450 °C erfolgte ein langsames Aufheizen, wobei als bevorzugte Aufheizrate 3 K/min gewählt wurde. Nach Erreichen der Temperatur $T_2$, die bei ca. 600 °C lag, wurden die Grünlinge über die Zeit $t_1$ von in etwa 10 min gehalten. Diese Parameter reichten grundsätzlich aus, damit der Binder entfernt wurde.

Zur Herstellung eines CoCrMo-Rohlings wurde sodann ein entbinderter Grünling vorgesintert. Hierzu wurde nach einer Alternative der Grünling auf eine Temperatur im Bereich von in etwa 800 °C schnell aufgeheizt (Aufheizrate im Bereich von 90 K/min) und bei dieser Temperatur über einen Zeitraum von in etwa 20 min gehalten. Anschließend erfolgte ein Kühlen mit anfangs gleicher, in der Folge niedriger Rate.

Durch dieses Verfahren wurde den Beziehungen bzw. Bedingungen gemäß der Gleichungen (1), (2) und (3) entsprochen:

$$t = t_0 \cdot \ln\left(\frac{c_0}{c}\right) \cdot \exp\left(\frac{T_0}{T_{VS}}\right)$$

$$= 0{,}0125\,\text{min} \cdot \ln\left(\frac{0{,}20}{0{,}19}\right) \cdot \exp\left(\frac{11000\,K}{1073\,K}\right) \approx 18\,\text{min}$$

und somit $t/2 < t_{VS} < 2t$, hier 9 min $< t_{VS} <$ 36 min und

$$\frac{T_{VS} - 650°C}{R_{HVS}} + \frac{T_{VS} - 650°C}{R_{KVS}} < 2t$$

$$\text{hier}: \frac{800°C - 650°C}{90\,K/\text{min}} + \frac{800°C - 650°C}{90\,K/\text{min}} \approx 3{,}3\,\text{min} < 36\,\text{min}.$$

Schliffbilder so hergestellter Rohlinge zeigten, dass sich eine offene Flächenporosität im Bereich zwischen 16 % und 22 %, mit einer Häufung zwischen 18 % und 20 % ergab. Entsprechende Rohlinge waren einfach zu bearbeiten, ohne dass das Risiko eines hohen Werkzeugverschleißes erfolgte, wodurch die Genauigkeit der Bearbeitung Einbußen erfahren kann.

Durch die Flächenporosität war ein einfaches Bearbeiten an einer CAM-Maschine möglich. Hierzu wurde der Rohling mit einer Halterung in der CAM-Maschine fixiert. Anschließend erfolgte ein abtragendes Bearbeiten, wobei Bereiche des Rohlings in einem nassen und in einem trockenen System bearbeitet worden sind. Wurde trocken gefräst, wurde der beim Bearbeiten entstehende Staub mittels eines Klasse-H-Staubsaugers entfernt. Wurde ein Nassverfahren eingesetzt, so wurde schleifend gearbeitet. Insbesondere beim Nassbearbeiten zeigten sich keine Nachteile.

Die aus dem Rohling herausgearbeiteten Körper wiesen dabei eine Dimensionierung auf, die der beim Durchsintern erfolgenden Schrumpfung Rechnung trägt. Dabei konnte nach der Schrumpfung festgestellt werden, dass diese gleichmäßig und passungsgerecht erfolgte. Anschließend wurde die Oberfläche des Formkörpers poliert oder in gewohnter Weise eine keramische Verblendung aufgebracht, die absolut blasenfrei war, ohne dass die vorhandene Restflächenporosität zu Problemen führte.

Wurde die Erfindung anhand der Herstellung von Zahnersatz erläutert, so wird hierdurch die Erfindung nicht eingeschränkt.

## Patentansprüche

1. Verfahren zur Herstellung eines Formkörpers, insbesondere eines Zahnersatzes oder eines Teils von diesem, mit den Verfahrensschritten:

   - Herstellung einer Mischung aus einem Metallpulver in Form einer Nickel-Chrom-Legierung oder einer Kobalt-Chrom-Legierung und einem Bindemittel,
   - Verdichten der Mischung zu einem Grünling,
   - Aufheizen des Grünlings von Zimmertemperatur auf eine Entbinderungsstarttemperatur $T_1$,
   - Entbindern des Grünlings, wobei der Grünling zum Entbindern zunächst auf die Entbinderungsstarttemperatur $T_1$ mit 350 °C $\leq T_1 \leq$ 550 °C erhitzt und sodann im Temperaturbereich zwischen $T_1$ und der Entbinderungsend-

temperatur $T_2$ mit 550 °C < $T_2$ < 650 °C mit $T_2$ > $T_1$ bei einer Aufheizrate $R_1$ mit 1 K/min ≤ $R_1$ ≤ 5 K/min auf $T_2$ erhitzt wird,

- Vorsintern des entbinderten Grünlings, wobei der Grünling mit einer Aufheizrate $R_{HVS}$ mit 5 K/min ≤ $R_{HVS}$ ≤ 200 K/min auf eine Vorsinterendtemperatur $T_{VS}$ mit 750 °C ≤ $T_{VS}$ < 850 °C erhitzt und über eine Haltezeit $t_{vs}$ mit 5 Min. ≤ $t_{vs}$ ≤ 60 Min. gehalten wird,

- Kühlen des Grünlings von der Vorsinterendtemperatur $T_{VS}$ mit einer Kühlrate $R_{KVS}$ mit 5 K/min ≤ $R_{KVS}$ ≤ 200 K/min, wobei zumindest die Aufheizrate $R_{HVS}$, die Vorsinterendtemperatur $T_{VS}$ und die Kühlrate $R_{KVS}$ derart aufeinander abgestimmt werden, dass der vorgesinterte, einen Rohling bildende Grünling eine Flächenporosität zwischen 16 % und 22 % nach dem Vorsintern aufweist,

- abtragendes Bearbeiten des Rohlings und

- Dichtsintern des bearbeiteten Rohlings zur Bildung des Formkörpers.

2. Verfahren nach Anspruch 1,
   **dadurch gekennzeichnet,**
   **dass** der Grünling nach Halten auf der Vorsinterendtemperatur Tvs mit der Kühlrate $R_{KVS}$ auf eine Temperatur $T_3$ abgekühlt wird mit 450 °C ≤ $T_3$ ≤ 650 °C.

3. Verfahren nach Anspruch 1 oder Anspruch 2,
   **dadurch gekennzeichnet,**
   **dass** der Grünling bei der Entbinderungsendtemperatur $T_2$ über eine Zeit $t_2$ mit 1 min ≤ $t_2$ ≤ 20 min gehalten wird.

4. Verfahren nach zumindest einem der Ansprüche 1 bis 3,
   **dadurch gekennzeichnet,**
   **dass** als der verdichtete Grünling ein solcher verwendet wird, dessen Flächenporosität zwischen 16 % und 27 %, insbesondere zwischen 18 % und 22 % liegt.

5. Verfahren nach Anspruch 1,
   **dadurch gekennzeichnet,**
   **dass** bei einer Kobalt-Chrom-Legierung folgende Zusammensetzung verwendet wird:

| | |
|---|---|
| Kobalt: | 50 Gew.-% bis 70 Gew.-% |
| Chrom: | 20 Gew.-% bis 35 Gew.-% |
| Molybdän: | 0 Gew.-% bis 10 Gew.-% |
| Wolfram: | 0 Gew.-% bis 20 Gew.-% |
| weitere Elemente: | weniger als 10 Gew.-%, |

wobei die Gesamtsumme 100 Gew.-% beträgt,
und bei einer Nickel-Chrom-Legierung folgende Zusammensetzung verwendet wird:

| | |
|---|---|
| Nickel: | 50 Gew.-% bis 70 Gew.-% |
| Chrom: | 20 Gew.-% bis 35 Gew.-% |
| Molybdän: | 0 Gew.-% bis 10 Gew.-% |
| Wolfram: | 0 Gew.-% bis 20 Gew.-% |
| weitere Elemente: | weniger als 10 Gew.-%, |

mit einer Gesamtsumme von 100 Gew.-%.

6. Verfahren nach zumindest einem der Ansprüche 1 bis 5,
   **dadurch gekennzeichnet,**
   **dass** der Grünling bei der Vorsinterendtemperatur $T_{VS}$ über eine Haltezeit $t_{VS}$ gehalten wird, wobei je höher $T_{VS}$ gewählt wird, je kürzer die Haltezeit $t_{VS}$ eingestellt wird.

7. Verfahren nach zumindest einem der Ansprüche 1 bis 6,
   **dadurch gekennzeichnet,**
   **dass** bei der Vorsinterendtemperatur $T_{VS}$ die Haltezeit $t_{VS}$ mit 10 min ≤ $t_{vs}$ ≤ 30 min eingestellt wird und vorzugsweise die Aufheizrate $R_{HVS}$ und die Kühlrate $R_{KVS}$ eingestellt werden auf 5 K/min ≤ $R_{HVS}$ ≤ 20 K/min bzw. 5 K/min ≤ $R_{KVS}$

$\leq$ 20 K/min.

8.  Verfahren nach zumindest einem der vorhergehenden Ansprüche,
    **dadurch gekennzeichnet,**
    **dass** der Grünling bei der Vorsinterendtemperatur $T_{VS}$ in Kelvin, über eine Zeit $t_{VS}$ gehalten wird mit

$$t/2 < t_{VS} < 2t$$

mit:

$$t = t_0 \cdot \ln\!\left(\frac{c_0}{c}\right) \cdot \exp\!\left(\frac{T_0}{T_{VS}}\right)$$

mit

c = Gewünschter Flächenporositätsanteil des Grünlings nach dem Vorsintern mit 16 % < c < 22 %, insbesondere 18 % < c < 20 %,
$c_0$ = Flächenporositätsanteil des Grünling nach dem Entbindern mit $c_0$ - c < 5 %, vorzugsweise $c_0$ - c < 2 %, mit $c_0$ > c.
to = Materialkonstante mit $t_0$=0,0125 min
$T_0$ = Materialkonstante mit $T_0$=11000 K.
$T_{VS}$ = Vorsinterendtemperatur bei der Haltezeit $t_{vs}$ mit 650 °C $\leq T_{VS} \leq$ 1100 °C.

9.  Verfahren nach Anspruch 8,
    **dadurch gekennzeichnet,**
    **dass** die Aufheizzeit mit annähernd konstanter Aufheizrate $R_{HVS}$ und die Kühlzeit mit annähernd konstanter Kühlrate $R_{KVS}$ im Bereich zwischen 650 °C und der Vorsinterendtemperatur $T_{VS}$, angegeben in Grad Celsisus, derart eingestellt werden, dass deren Gesamtsumme der Bedingung genügt:

$$\frac{T_{VS} - 650°C}{R_{HVS}} + \frac{T_{VS} - 650°C}{R_{KVS}} < 2t$$

wobei

$$t = t_0 \cdot \ln\!\left(\frac{c_0}{c}\right) \cdot \exp\!\left(\frac{T_0}{T_{VS}}\right)$$

und Tvs und $c_0$ - c derart aufeinander abgestimmt werden, dass negative Aufheiz- und Kühlraten ausgeschlossen sind.

10. Verfahren nach zumindest einem der vorhergehenden Ansprüche,
    **dadurch gekennzeichnet,**
    **dass** die Metalllegierung zu Pulver verdüst und mit einem Bindemittel auf Wachs- und/oder Zellulose-Basis zur Herstellung der Mischung vermischt wird, dass bevorzugterweise der Grünkörper durch isostatisches oder axiales Pressen der aus dem Metallpulver und dem Bindemittel bestehenden Mischung bei einem Druck p mit 100 MPa $\leq$ p $\leq$ 1.000 MPa, insbesondere 200 MPa $\leq$ P $\leq$ 600 MPa, oder durch Metallpulverspritzgießverfahren hergestellt wird und dass bevorzugterweise das Entbindern und Vorsintern und vorzugsweise das Kühlen unter Sauerstoffabschluss, insbesondere in Inert- oder reduzierender Atmosphäre wie Formiergas-Atmosphäre oder bei Vakuum durchgeführt wird.

**Claims**

1. Method for the manufacture of a moulded body, in particular a dental prosthesis or a part thereof, characterised through the following steps:

   - Production of a mixture of a metal powder in the form of a nickel-chromium alloy or a cobalt-chromium alloy and a binding agent,
   - Compression of the mixture to form a green body,
   - Heating of the green body from room temperature to a debinding start temperature $T_1$,
   - Debinding of the green body, wherein for debinding purposes the green body is first heated to the debinding start temperature $T_1$ whereby 350 °C $\leq T_1 \leq$ 550 °C and is then heated in the temperature range between $T_1$ and the debinding end temperature $T_2$ whereby 550 °C < $T_2$ < 650 °C whereby $T_2 > T_1$ at a heating rate $R_1$, whereby 1 K/min $\leq R_1 \leq$ 5 K/min,
   - Pre-sintering of the green body after debinding, wherein the green body is heated at a heating rate $R_{HVS}$ of 5 K/min $\leq R_{HVS} \leq$ 200 K/min to a pre-sintering end temperature Tvs whereby 750 °C $\leq$ Tvs < 850 °C and is maintained for a holding period $t_{vs}$ of 5 min $\leq t_{vs} \leq$ 60 minutes,
   - Cooling of the green body from the pre-sintering end temperature Tvs at a cooling rate $R_{KVS}$ whereby 5 K/min $\leq R_{KVS}$ < 200 K/min, wherein at least the heating rate $R_{HVS}$, the pre-sintering end temperature Tvs and the cooling rate $R_{KVS}$ are matched to one another such that the pre-sintered green body forming a blank has a surface porosity between 16 % and 22 % after pre-sintering,
   - Material-removing working of the blank and
   - Sintering of the worked blank to full density to form the moulding.

2. Method according to claim 1,
   **characterised in that**
   after maintenance at the pre-sintering end temperature Tvs the green body is cooled at the cooling rate $R_{KVS}$ to a temperature $T_3$ whereby 450 °C $\leq T_3 \leq$ 650 °C.

3. Method according to claim 1 or claim 2,
   **characterised in that**
   the green body is maintained at the debinding end temperature $T_2$ over a period of time $t_2$ whereby 1 min $\leq t_2 \leq$ 20 min.

4. Method according to at least one of claims 1 to 3,
   **characterized in that**
   the compacted green compact used is a green compact with a surface porosity between 16 % and 27 %, in particular between 18 % and 22 %.

5. Method of claim 1,
   **characterized in that**
   for a cobalt-chromium alloy one chooses the following composition:

   | | |
   |---|---|
   | Cobalt: | 50% to 70% by weight |
   | Chromium: | 20 % to 35 % by weight |
   | Molybdenum: | 0 % to 10% by weight |
   | Tungsten: | 0 % to 20% by weight |
   | Other elements: | less than 10% by weight, |

   whereby the sum total adds up to 100% by weight
   and for a nickel-chromium alloy one uses the following composition:

   | | |
   |---|---|
   | Nickel: | 50 % to 70 % by weight |
   | Chromium: | 20 % to 35 % by weight |
   | Molybdenum: | 0 % to 10 % by weight |
   | Tungsten: | 0% to 20 % by weight |
   | Other elements: | less than 10% by weight |

with a sum total of 100% by weight.

6.  Method according to at least one of claims 1 to 5,
    **characterised in that**
    the green body is maintained at the pre-sintering end temperature Tvs for a holding period $t_{VS}$, wherein the higher the value of $T_{VS}$ chosen the shorter the holding time $t_{VS}$.

7.  Method according to at least one of claims 1 to 6,
    **characterised in that**
    at the pre-sintering end temperature $T_{VS}$ the holding time tvs is set to 10 min $\leq t_{vs} \leq$ 30 min and the heating rate $R_{HVS}$ and the cooling rate $R_{KVS}$ are preferably set to 5 K/min $\leq R_{HVS} <$ 20 K/min and 5 K/min $\leq R_{KVS} <$ 20 K/min respectively.

8.  Method according to at least one of the preceding claims,
    **characterized in that**
    the green compact is kept at a presinter end temperature (tvs specified in K) for a time period tvs whereby

$$t/2 < tvs < 2t$$

with:

$$t = t_0 \cdot \ln\left(\frac{c_0}{c}\right) \cdot \exp\left(\frac{T_0}{T_{VS}}\right)$$

with

c = Desired surface porosity fraction of the green compact after presintering with 16 % < c < 22 %, in particular 18 % < c < 20 %,
$c_0$ = surface porosity fraction of the green compact after debinding with $c_0$ - c < 5 %, preferably $c_0$ - c < 2 %, in particular with $c_0$ > c.
$t_0$ = matter constnt with $t_0$=0.0125 min
$T_0$ = matter constant with $T_0$=11000 K.
$T_{VS}$ = presinter end temperature for a holding time $t_{vs}$ with 650 °C $\leq T_{VS} \leq$ 1100 °C.

9.  Method of claim 8,
    **characterized in that**
    the heating time with an approximately constant heat-up rate $R_{HVS}$ and the cooling time with an approximately constant cool-down rate $R_{KVS}$ are set within the range between 650 °C and the presinter end temperature Tvs, specified in degree Celsius, in a way so that they satisfy the condition:

$$\frac{T_{VS} - 650°C}{R_{HVS}} + \frac{T_{VS} - 650°C}{R_{KVS}} < 2t$$

whereby

$$t = t_0 \cdot \ln\left(\frac{c_0}{c}\right) \cdot \exp\left(\frac{T_0}{T_{VS}}\right)$$

and Tvs and $c_0$ - c are tuned relative to each other so that negative heat-up and cool-down times are ruled out.

10. Method according to at least one of the preceding claims,

**characterized in that**

the metal alloy is atomized into a powder and is mixed with a wax- and/or cellulose-based binding agent to produce a mixture, that preferably the green compact is produced by way of isostatic or axial pressing of the mixture consisting of the metal powder and the binding agent, at a pressure p with 100 MPa ≤ p ≤ 1,000 MPa, in particular 200 MPa ≤ p ≤ 600 MPa, or using a metal powder injection moulding process, and that preferably the debinding and presintering and preferably the cooling is performed in the absence of oxygen, in particular under an inert-gas atmosphere or a reducing atmosphere such as a forming gas atmosphere or in vacuum.

**Revendications**

1. Procédé de fabrication d'une pièce moulée, notamment d'une prothèse dentaire ou d'une partie de celle-ci, **caractérisé par**
   les étapes de procédé suivantes :

   - fabrication d'un mélange à partir d'une poudre métallique sous la forme d'un alliage nickel-chrome ou d'un alliage cobalt-chrome et d'un liant,
   - compression du mélange pour former une ébauche,
   - chauffe de l'ébauche de la température ambiante à une température de début du déliantage $T_1$,
   - déliantage de l'ébauche, sachant que l'ébauche à délianter est chauffée tout d'abord à la température de début du déliantage $T_1$ telle que 350 °C ≤ $T_1$ ≤ 550 °C, puis dans la plage de température comprise entre $T_1$ et la température de fin de déliantage $T_2$ telle que 550 °C < $T_2$ < 650 °C où $T_2$ > $T_1$, chauffée à la température $T_2$ à une vitesse de chauffage $R_1$ telle que 1 K/min ≤ $R_1$ ≤ 5 K/min,
   - pré-frittage de l'ébauche déliantée, sachant que l'ébauche est chauffée à une température de fin de pré-frittage Tvs telle que 750 °C ≤ $T_{VS}$ < 850 °C à une vitesse de chauffage $R_{HVS}$ telle que 5 K/min ≤ $R_{HVS}$ < 200 K/min et maintenue à température pendant une durée de maintien $t_{VS}$ telle que 5 min ≤ $t_{vs}$ ≤ 60 min.
   - refroidissement de l'ébauche depuis la température de fin de pré-frittage $T_{VS}$ à une vitesse de refroidissement $R_{KVS}$ telle que 5 K/min ≤ $R_{KVS}$ < 200 K/min, sachant qu'au moins la vitesse de chauffage $R_{HVS}$, la température de fin de pré-frittage Tvs et la vitesse de refroidissement $R_{KVS}$ sont harmonisées entre elles de manière à ce que l'ébauche préfrittée formant une pièce brute présente après le pré-frittage une porosité surfacique comprise entre 16 % et 22 %.
   - usinage par enlèvement de matière de la pièce brute et
   - frittage à densité maximale de la pièce brute usinée pour former la pièce moulée.

2. Procédé selon la revendication 1,
   **caractérisé en ce**
   **que** l'ébauche une fois maintenue à la température de fin de pré-frittage Tvs est refroidie à une température $T_3$ telle que 450 °C ≤ $T_3$ ≤ 650 °C à la vitesse de refroidissement $R_{KVS}$.

3. Procédé selon la revendication 1 ou 2,
   **caractérisé en ce**
   **que** l'ébauche est maintenue à la température de fin de déliantage $T_2$ pendant une durée $t_2$ telle que 1 min ≤ $t_2$ ≤ 20 min.

4. Procédé selon au moins une des revendications 1 à 3,
   **caractérisé en ce**
   **qu'**est utilisée comme ébauche comprimée une ébauche dont la porosité surfacique est comprise entre 16 % et 27 %, notamment entre 18 % et 22 %.

5. Procédé selon la revendication 1,
   **caractérisé en ce**
   **que** la composition suivante est utilisée pour un alliage cobalt-chrome :

   | | |
   |---|---|
   | cobalt : | 50 % en poids à 70 % en poids |
   | chrome : | 20 % en poids à 35 % en poids |
   | molybdène : | 0 % en poids à 10 % en poids |
   | tungstène : | 0 % en poids à 20 % en poids |
   | autres éléments : | moins de 10 % en poids |

sachant que la somme totale s'élève à 100 % en poids,
et **que** la composition suivante est utilisée pour un alliage nickel-chrome :

| | |
|---|---|
| nickel : | 50 % en poids à 70 % en poids |
| chrome : | 20 % en poids à 35 % en poids |
| molybdène : | 0 % en poids à 10 % en poids |
| tungstène : | 0 % en poids à 20 % en poids |
| autres éléments : | moins de 10 % en poids |

avec une somme totale de 100 % en poids.

6. Procédé selon au moins une des revendications 1 à 5,
   **caractérisé en ce**
   **que** l'ébauche est maintenue à la température de fin de pré-frittage Tvs pendant une durée de maintien tvs, sachant que plus la valeur choisie pour Tvs est élevée, plus la durée de maintien tvs est courte.

7. Procédé selon au moins une des revendications 1 à 6,
   **caractérisé en ce**
   **qu'**est réglée, à la température de fin de pré-frittage $T_{VS}$, la durée de maintien $T_{vs}$ telle que 10 min ≤ $t_{vs}$ ≤ 30 min et que sont réglées de préférence la vitesse de chauffage $R_{HVS}$ et la vitesse de refroidissement $R_{KVS}$ sur respectivement 5 K/min ≤ $R_{HVS}$ < 20 K/min et 5 K/min ≤ $R_{KVS}$ < 20 K/min.

8. Procédé selon au moins une des revendications précédentes,
   **caractérisé en ce**
   **que** l'ébauche est maintenue pendant une durée tvs à la température de fin de pré-frittage Tvs en kelvins, telle que

$$t/2 < t_{VS} < 2t$$

où :

$$t = t_0 \cdot \ln\left(\frac{c_0}{c}\right) \cdot \exp\left(\frac{T_0}{T_{VS}}\right)$$

où

   c = part de porosité surfacique souhaitée de l'ébauche après le pré-frittage avec 16 % < c < 22 %, en particulier 18 % < c < 20 %,
   $c_0$ = part de porosité surfacique de l'ébauche après le déliantage telle que $c_0$ - c < 5 %, de préférence $c_0$ - c < 2 %, avec $c_0$ > c.
   $t_0$ = constante propre au matériau avec $t_0$=0,0125 min
   $t_0$ = constante propre au matériau avec $T_0$=11000 K.
   Tvs = température de fin de pré-frittage pour la durée de maintien $t_{vs}$ telle que 650 °C ≤ $T_{VS}$ ≤ 1100 °C.

9. Procédé selon la revendication 8,
   **caractérisé en ce**
   **que** la durée de chauffage avec une vitesse de chauffage presque constante $R_{HVS}$ et la durée de refroidissement avec une vitesse de refroidissement presque constante $R_{KVS}$ dans la plage comprise entre 650 °C et la température de fin de pré-frittage indiquée en degrés Celsius sont réglées de telle sorte que leur somme totale satisfait à la condition suivante :

$$\frac{T_{VS} - 650°C}{R_{HVS}} + \frac{T_{VS} - 650°C}{R_{KVS}} < 2t$$

sachant que

$$t = t_0 \cdot \ln\left(\frac{c_0}{c}\right) \cdot \exp\left(\frac{T_0}{T_{VS}}\right)$$

et Tvs et $c_0$ - c sont harmonisées entre elles de sorte que des vitesses de chauffage et de refroidissement négatives sont exclues.

10. Procédé selon au moins une des revendications précédentes,
**caractérisé en ce**
**que** l'alliage métallique est atomisé pour former une poudre et mélangé à un liant à base de cire et/ou de cellulose pour fabriquer le mélange, que de préférence l'ébauche est fabriquée par compression isostatique ou axiale du mélange constitué de la poudre métallique et du liant à une pression p telle que 100 MPa $\leq$ p $\leq$ 1000 MPa, en particulier 200 MPa $\leq$ P $\leq$ 600 MPa, ou par procédé de moulage par injection et que de préférence le déliantage et le pré-frittage et de préférence le refroidissement sont réalisés en l'absence d'oxygène, notamment sous atmosphère inerte ou réductrice telle qu'une atmosphère sous gaz de formage ou sous vide.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1067880 B
- DE 19938144 C
- WO 2009120749 A
- EP 1764062 A
- DE 10352231 A
- US 20050023710 A
- US 4996022 A
- AT 505698 A

**In der Beschreibung aufgeführte Nicht-Patentliteratur**

- **RODRIGUES et al.** Powder metallurgical Processing of Co-28%Cr-6%Mo for dental implants: Physical, cheanical and electrochemical properties. *Powder Technology,* 2011, vol. 2006, 233-238